(19) Europäisches Patentamt / European Patent Office / Office européen des brevets

(11) **EP 3 178 380 A1**

(12) **EUROPEAN PATENT APPLICATION**

(43) Date of publication:
**14.06.2017 Bulletin 2017/24**

(51) Int Cl.:
*A61B 5/00* (2006.01)

(21) Application number: **16201784.2**

(22) Date of filing: **01.12.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **09.12.2015 JP 2015240581**

(71) Applicant: **Canon Kabushiki Kaisha**
**Tokyo 146-8501 (JP)**

(72) Inventors:
• **FUKUTANI, Kazuhiko**
**Ohta-ku, Tokyo 146-8501 (JP)**
• **NANAUMI, Ryuichi**
**Ohta-ku, Tokyo 146-8501 (JP)**
• **MIYASATO, Takuro**
**Ohta-ku, Tokyo 146-8501 (JP)**
• **SHIRONO, Jumpei**
**Ohta-ku, Tokyo 146-8501 (JP)**
• **NAKANO, Kota**
**Ohta-ku, Tokyo 146-8501 (JP)**

(74) Representative: **Houle, Timothy James**
**Canon Europe Ltd**
**European Patent Department**
**3 The Square**
**Stockley Park**
**Uxbridge, Middlesex UB11 1ET (GB)**

(54) **PHOTOACOUSTIC APPARATUS, DISPLAY CONTROL METHOD, AND PROGRAM**

(57) A photoacoustic apparatus includes a light irradiation unit configured to irradiate a subject with light, a reception unit configured to convert an acoustic wave generated from the subject due to the irradiation with the light into an electric signal, a processing unit configured to acquire image data using the electric signal, and a display control unit. The processing unit acquires first image data by a first image generation method using the electric signal, and acquires second image data by a second image generation method different from the first image generation method using the electric signal. The display control unit sets, as a difference region, a region in which difference image data between the first image data and the second image data has an image value within a predetermined numerical range, and causes image data in which the difference region and another region are distinguishable from each other to be displayed on a display unit using the first image data and the second image data.

**FIG.1**

EP 3 178 380 A1

**Description**

BACKGROUND OF THE INVENTION

Field of the Invention

The present invention relates to a photoacoustic apparatus that acquires image data derived from a photoacoustic wave generated by irradiating a subject with light.

Description of the Related Art

There is a technique called photoacoustic imaging (PAI) as a technique for acquiring subject information based on an acoustic wave from a subject. The photoacoustic imaging is a technique that images the inside of the subject with use of a photoacoustic effect, in which irradiation of the subject with pulsed light causes the acoustic wave (photoacoustic wave) to be generated due to absorption of the light inside the subject.

It is known that, in the photoacoustic imaging, typically, an analytical image reconstruction method, such as the filtered back-projection method and the Fourier transform method, cannot completely reproduce a shape of a source from which the acoustic wave is generated, if a region where the acoustic wave is measured is insufficient for a region to be imaged. For example, the region where the acoustic wave is measured can be said to be insufficient if the acoustic wave can be received only from a certain specific direction not from every direction around the subject (called a "Limited View condition").

As a method for solving the above-described problem, Japanese Patent Application Laid-Open No. 2014-180492 discusses that image data is generated based on information regarding a structure of the subject (characteristic information derived from a characteristic structure of the subject). According to this method, the shape of the acoustic source from which the photoacoustic wave is generated can be further faithfully reproduced even under the Limited View condition.

SUMMARY OF THE INVENTION

The method discussed in Japanese Patent Application Laid-Open No. 2014-180492 involves such a risk that image data indicating a different structure from the actual structure may be generated when the structural characteristic of the source, from which the acoustic wave is generated, is different from the information regarding the structure of the subject used in the generation of the image data. In other words, the image data acquired by the method discussed in Japanese Patent Application Laid-Open No. 2014-180492 may include both a highly reliable region (an image region that matches the actual structure) and a low reliability region (an image region that indicates the different structure from the actual structure).

The present invention is directed to a photoacoustic apparatus capable of displaying the image data so as to allow a user to distinguish the low reliability region in the image data.

According to a first aspect of the present invention, there is provided photoacoustic apparatus as specified in claims 1 to 12. According to a second aspect of the present invention, there is provided a display control method as specified in clams 13 and 14. According to a third aspect of the present invention, there is provided a program as specified in clam 15.

Further features of the present invention will become apparent from the following description of exemplary embodiments with reference to the attached drawings.

BRIEF DESCRIPTION OF THE DRAWINGS

Fig. 1 is a schematic diagram illustrating a photoacoustic apparatus according to a first exemplary embodiment.
Fig. 2 is a block diagram illustrating a specific example of a computer according to the first exemplary embodiment.
Fig. 3 is a flowchart illustrating signal processing according to the first exemplary embodiment.
Fig. 4 illustrates a simulation model according to the first exemplary embodiment.
Figs. 5A, 5B, 5C, and 5D illustrate a model diagram and image data of an optical absorber according to the first exemplary embodiment.
Figs. 6A, 6B, and 6C each illustrate a graphical user interface (GUI) according to the first exemplary embodiment.
Fig. 7 illustrates a vascular model according to a second exemplary embodiment.
Figs. 8A, 8B, and 8C each illustrate a GUI according to the second exemplary embodiment.

DESCRIPTION OF THE EMBODIMENTS

Hereinbelow, exemplary embodiments of the present invention will be described in further detail with reference to the drawings. The same components will be identified by the same reference numerals in principle, and redundant descriptions thereof will be omitted. Each of the embodiments of the present invention described below can be implemented solely or as a combination of a plurality of the embodiments or features thereof where necessary or where the combination of elements or features from individual embodiments in a single embodiment is beneficial.

<Basic Configuration>

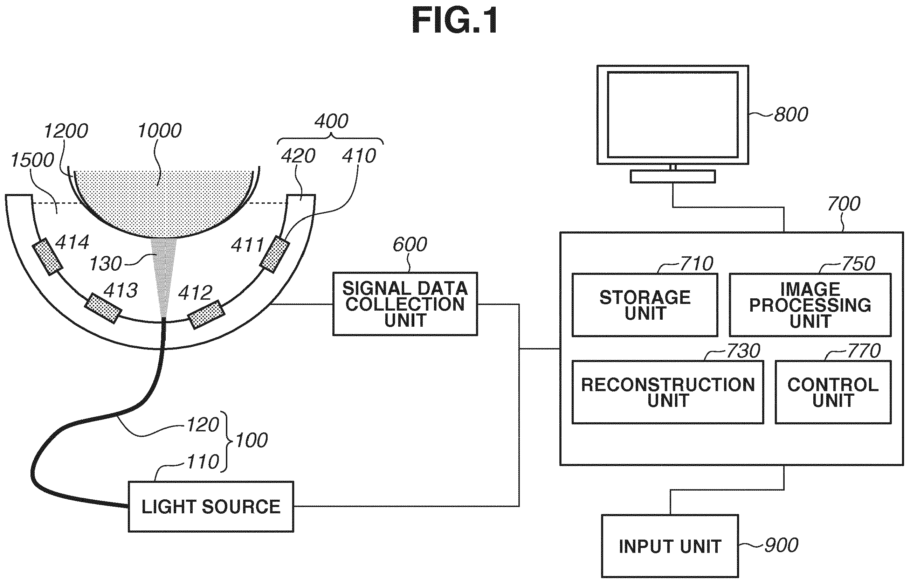

A configuration of a photoacoustic apparatus as a subject information acquisition apparatus according to a first exemplary embodiment of the present invention will be described with reference to Fig. 1. Fig. 1 is a schematic diagram illustrating the photoacoustic apparatus according to the present exemplary embodiment. The photoacoustic apparatus includes a light irradiation unit 100, a reception unit 400, a signal data collection unit 600, a computer 700, a display unit 800, an input unit 900, and a holding unit 1200. A measurement target is a subject 1000.

The light irradiation unit 100 irradiates the subject 1000 with pulsed light 130, and an acoustic wave is generated in the subject 1000. The acoustic wave generated due to the light and based on the photoacoustic effect will be referred to as a photoacoustic wave. The reception unit 400 outputs an electric signal as an analog signal by receiving the photoacoustic wave. The signal data collection unit 600 converts the electric signal as the analog signal output from the reception unit 400 into a digital signal, and outputs the converted signal to the computer 700. The computer 700 stores the digital signal output from the signal data collection unit 600 as signal data derived from the photoacoustic wave. A process from the irradiation with the light to the output of the digital signal to be stored as the signal data will be referred to as "photoacoustic measurement".

The computer 700 generates image data indicating information regarding the subject 1000 (subject information) by performing signal processing on the stored digital signal. Further, the computer 700 outputs the image data to the display unit 800 after performing image processing on the acquired image data. An image of the information regarding the subject 1000 is displayed on the display unit 800. A doctor as a user can make a diagnosis by checking the image of the information regarding the subject 1000 that is displayed on the display unit 800.

The subject information acquired from the photoacoustic apparatus according to the present exemplary embodiment is at least one of, for example, pieces of information regarding a generated acoustic pressure of the photoacoustic wave (initial acoustic pressure), a density of optical absorption energy, an optical absorption coefficient, and a concentration of a substance forming the subject 1000. The information regarding the concentration of the substance is, for example, a concentration of oxyhemoglobin, a concentration of deoxyhemoglobin, a total concentration of hemoglobin, oxygen saturation. The total concentration of hemoglobin corresponds to a sum of the concentration of oxyhemoglobin and the concentration of deoxyhemoglobin. The oxygen saturation corresponds to a ratio of oxyhemoglobin to a total amount of hemoglobin. The photoacoustic apparatus according to the present exemplary embodiment acquires image data indicating a value of the above-described information at each position (each position in a two-dimensional or three-dimensional space) in the subject 1000.

Now, each of the units of the photoacoustic apparatus according to the present exemplary embodiment will be described.

<Light Irradiation Unit 100>

The light irradiation unit 100 includes a light source 110, which emits the pulsed light 130, and an optical system 120, which guides the pulsed light 130 emitted from the light source 1 to the subject 1000.

A pulse width of the light 130 emitted by the light source 110 may be a pulse width of 1 ns or longer and 100 ns or shorter. Further, a wavelength of the light 130 may be a wavelength that falls within a range approximately from 400 nm to 1600 nm. In a case where a blood vessel located close to a surface of a living body is imaged at a high resolution, the wavelength of the light 130 may be set to a wavelength largely absorbable by the blood vessel (400 nm or longer and 700 nm or shorter). On the other hand, in a case where a deep portion in the living body is imaged, the photoacoustic apparatus may employ light having a wavelength (700 nm or longer and 1100 nm or shorter) typically little absorbable at a background tissue of the living body (e.g., water, fat).

A laser or a light-emitting diode can be used as the light source 110. Further, in a case where the subject 1000 is measured with use of light beams respectively having a plurality of wavelengths, the light source 110 may be a light source capable of converting the wavelength. Another possible configuration in the case where the subject 1000 is irradiated with a plurality of wavelengths is to prepare a plurality of light sources that respectively generates light beams having wavelengths different from each other, and irradiate the subject 1000 from each of the light sources alternately or in turn. Even when the plurality of light sources is used, they will be collectively expressed as the light source. Various types of lasers, such as a solid-state laser, a gas laser, a dye laser, and a semiconductor laser, can be used as the laser. For example, a pulse laser, such as a neodymium-doped yttrium aluminum garnet (Nd:YAG) laser and an alexandrite laser, may be used as the light source 110. Alternatively, a titanium sapphire (Ti:sa) laser using Nd:YAG laser light as excitation light, and an optical parametric oscillator (OPO) laser may be used as the light source 110.

An optical element such as a lens, a mirror, and an optical fiber can be used as the optical system 120. In a case where a breast or the like is the subject 1000, it is desirable to irradiate the subject 1000 while expanding a beam diameter of the pulsed light 130, whereby a light emission portion of the optical system 120 may be formed by a diffusing plate or the like that diffuses the light 130. On the other hand, in a case where the photoacoustic apparatus functions as a photoacoustic microscope, the light emission portion of the optical system 120 may be formed by a lens or the like to allow the subject 1000 to be irradiated while the beam is focused thereon so as to increase resolution.

The subject 1000 may be irradiated with the pulsed light 130 directly from the light source 110 with the light irradiation unit 100 unequipped with the optical system 120.

<Reception Unit 400>

The reception unit 400 includes a reception element group 410 including reception elements 411 to 414, each of which outputs the electric signal by receiving the acoustic wave, and a support member 420, which supports the reception element group 410.

A piezoelectric ceramic material represented by lead zirconate titanate (PZT), a polymer piezoelectric film material represented by polyvinylidene fluoride (PVDF), and the like can be used as a member forming each of the reception elements 411 to 414. Alternatively, an element other than the piezoelectric element may be used. For example, an electrostatic capacitance type transducer (e.g., capacitive micro-machined ultrasonic transducer (CMUT)), and a transducer using a Fabry-Perot interferometer can be used. Any transducer may be employed as each of the reception elements 411 to 414 as long as this transducer can output the electric signal by receiving the acoustic wave. Further, the signal acquired by each of the reception elements 411 to 414 is a time-resolved signal. In other words, an amplitude of the signal acquired by each of the reception elements 411 to 414 indicates a value based on the acoustic pressure (e.g., a value proportional to the acoustic pressure) received by each of the reception elements 411 to 414 at each time.

The support member 420 may be made from, for example, a metallic material having high mechanical strength. In the present exemplary embodiment, the support member 420 is shaped like a semispherical shell and is configured to be able to support the reception element group 410 on the semispherical shell. In this case, respective directional axes of the reception elements 411 to 414 are collected to around a center of a curvature of the semi-sphere. Then, an image quality increases at around the center of the curvature when the subject 1000 is imaged with use of a group of electric signals output from these reception elements 411 to 414. The support member 420 may be configured in any manner as long as the support member 420 can support the reception element group 410. The support member 420 may be configured in such a manner that a plurality of reception elements is disposed so as to be arrayed on a flat surface or a curved surface like an array called a 1-dimensional (D) array, a 1.5-D array, a 1.75-D array, or a 2-D array.

Further, in the present exemplary embodiment, the support member 420 functions as a container that stores acoustic matching liquid 1500.

Further, the reception unit 400 may include an amplifier that amplifies the time series analog signal output from each of the reception elements 411 to 414. Further, the reception unit 400 may include an analog-to-digital (A/D) converter that converts the time series analog signal output from each of the reception elements 411 to 414 into a time series digital signal. In the other words, the reception unit 400 may include the signal data collection unit 600.

Ideally, it is desirable that the reception element group 410 is arranged so as to surround the subject 1000 from all around the subject 1000 to allow the acoustic wave to be detected from various angles. However, in a case where the reception elements 411 to 414 cannot be arranged so as to broadly surround the subject 1000 from all around the subject 1000 because the subject 1000 is large, the reception element group 410 may be arranged on the semispherical support member 420 to make the layout thereof more resembling a state surrounding the subject 1000 from all around the subject 1000 as illustrated in Fig. 1.

The layout and the number of the reception elements 411 to 414, and the shape of the support member 420 can be selected differently as long as they are optimized according to the subject 1000, and any kind of reception unit 400 may be employed regarding the present invention.

<Signal Data Collection Unit 600>

The signal data collection unit 600 includes an amplifier that amplifies the electric signal as the analog signal output from each of the reception elements 411 to 414, and an A/D converter that converts the analog signal output from the amplifier into the digital signal. The signal data collection unit 600 may be embodied with use of a field programmable gate array (FPGA) chip or the like. The digital signal output from the signal data collection unit 600 is stored into a storage unit 710 in the computer 700. The signal data collection unit 600 is also called a data acquisition system (DAS). The electric signal herein is a concept including both the analog signal and the digital signal. The signal data collection unit 600 may be connected to a photo-detection sensor mounted at the light emission portion of the light irradiation unit 100, and processing thereof may be started synchronously by being triggered by the emission of the pulsed light 130 from the light irradiation unit 100.

<Computer 700>

The computer 700 includes the storage unit 710, a reconstruction unit 730, an image processing unit 750, and a control unit 770. A function of each of the units will be described later at the time of a description of a processing flow.

The storage unit 710 can be embodied with use of a non-transitory storage medium, such as a read only memory (ROM), a magnetic disk, and a flash memory. Alternatively, the storage unit 710 may be a volatile medium, such as a random access memory (RAM). A storage medium storing a program is a non-transitory storage medium.

A unit in charge of a calculation function as a processing unit, such as the reconstruction unit 730 or the image processing unit 750, can be embodied with use of a processor, such as a central processing unit (CPU) and a graphics processing unit (GPU), or a calculation circuit, such as an FPGA chip. Not only these units may be embodied with use of a single processor or calculation circuit, but also these units may be embodied with use of a plurality of processors and/or calculation circuits.

The control unit 770 is embodied with use of a calculation element, such as a CPU. The control unit 770 controls an operation of each of the units of the photoacoustic apparatus. The control unit 770 may receive an instruction signal issued according to various kinds of operations such as a start of the measurement from the input unit 900, and control each of the units of the photoacoustic apparatus. Further, the control unit 770 reads out a program code stored in the storage unit 710, and controls activation of each of the units of the photoacoustic apparatus.

The computer 700 may be a workstation designed specifically therefor. Further, each of the units of the computer 700 may be embodied with use of a different hardware device. Alternatively, at least a part of the units of the computer 700 may be embodied with use of a single hardware device.

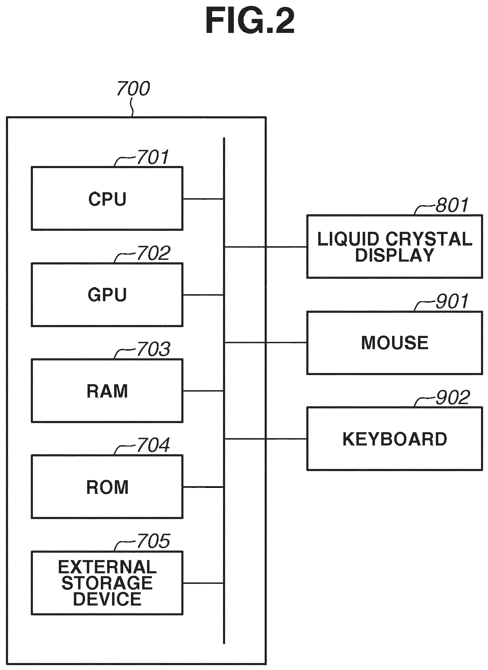

Fig. 2 is a block diagram illustrating a specific configuration of the computer 700 according to the present exemplary embodiment. The computer 700 according to the present exemplary embodiment includes a CPU 701, a GPU 702, a RAM 703, a ROM 704, and an external storage device 705. Further, a liquid crystal display 801 as the display unit 800, a mouse 901 and a keyboard 902 as the input unit 900 are connected to the computer 700.

<Display Unit 800>

The display unit 800 is a display such as a liquid crystal display and an organic electro luminescence (EL) display. The display unit 800 is a device that displays the image based on the subject information and the like, a numerical value at a specific position, and the like acquired by the computer 700. The display unit 800 may display a graphical user interface (GUI) for operating the image and the apparatus.

<Input Unit 900>

The input unit 900 can include a mouse and a keyboard operable by the user. Further, the display unit 800 may be constructed with use of a touch panel, thereby allowing the display unit 800 to serve as the input unit 900.

Each of the units of the photoacoustic apparatus may be configured as an individually different device, or may be configured as an integrated single device. Alternatively, at least a part of the units of the photoacoustic apparatus may be configured as an integrated single device.

<Subject 1000>

The subject 1000 will be described below, although being not a component forming the photoacoustic apparatus. The photoacoustic apparatus according to the present exemplary embodiment may be designed for the purpose of a diagnosis, a chemical treatment, follow-up monitoring, and the like of a malignant tumor, a vascular disease, and the like of a human and an animal. Therefore, a site targeted for the diagnosis, such as a living body, in particular, a breast, a neck, or an abdomen of a human or an animal, is expected to be handled as the subject 1000. For example, in a case where the measurement target is a human body, oxyhemoglobin, deoxyhemoglobin, a blood vessel including a large amount of them, a new blood vessel formed close to a tumor, or the like may be handled as an optical absorber target.

<Holding Unit 1200>

The holding unit 1200 is used to hold the shape of the subject 1000 during the measurement. Holding the subject 1000 with the holding unit 1200 can constrain a motion of the subject 1000 and keep a position of the subject 1000 within the holding unit 1200. Glycol-modified polyethylene terephthalate (PET-G) or the like can be used as a material of the holding unit 1200.

It is desirable that the holding unit 1200 is made from a material having hardness sufficient for the holding unit 1200 to hold the subject 1000. The holding unit 1200 may be made from a material that permits the light 130 for use in the measurement to be transmitted therethrough. The holding unit 1200 may be made from a material having similar impedance to the subject 1000. In a case where an object having a curved surface, such as the breast, is handled as the subject 1000, the holding unit 1200 may be formed into a concaved shape. In this case, the subject 1000 can be inserted in the concaved potion of the holding unit 1200.

In a case where the subject 1000 does not have to be held, the photoacoustic apparatus may be unequipped with the holding unit 1200.

<Acoustic Matching Liquid 1500>

The acoustic matching liquid 1500 will be described now, although being not a component forming the photoacoustic apparatus. The acoustic matching liquid 1500 serves to facilitate propagation of the acoustic wave between the holding unit 1200 and the reception elements 411 to 414. Water, ultrasonic gel, or the like may be used as the acoustic matching liquid 1500. The acoustic matching liquid 1500 may be liquid that little attenuates the acoustic wave. The acoustic matching liquid 1500 may be transparent for the irradiation light if the irradiation light is transmitted through the acoustic matching liquid 1500. A space between the subject 1000 and the holding unit 1200 is also filled with the acoustic matching liquid 1500.

In the present exemplary embodiment, the support member 420 also functions as a container that stores the acoustic matching liquid 1500 therein. The subject information acquisition apparatus may include a container capable of storing the acoustic matching liquid 1500 between the reception elements 411 to 414 and the subject 1000 as a different member from the support member 420.

The acoustic matching liquid 1500 may also be provided between the subject 1000 and the holding unit 1200. The description of the present exemplary embodiment will continue, assuming that acoustic matching is achieved between the subject 1000 and the holding unit 1200.

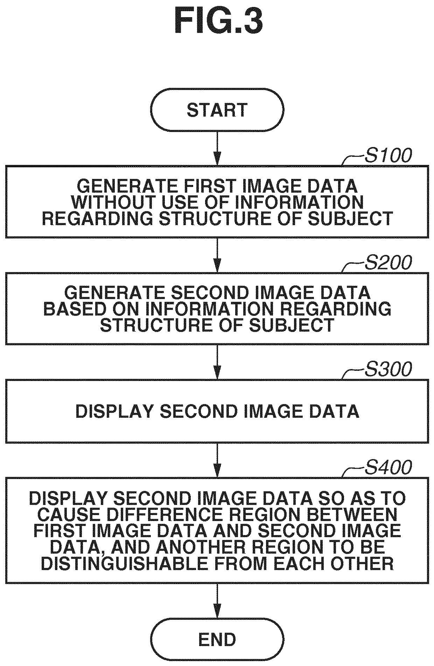

Next, a detail of a method for forming a photoacoustic image that is performed by the computer 700 will be described with reference to a flow illustrated in Fig. 3. The flowchart illustrated in Fig. 3 indicates a flow after the signal data derived from the electric signal output from the reception unit 400 is stored into the storage unit 710 of the computer 700.

<Step S100: Process for Generating Image Data without use of Information Regarding Structure of Subject 1000>

In this process, the computer 700 performs an image generation method for generating the image data without use of information regarding a structure of the subject 1000. This image generation method corresponds to a first image generation method in the present invention, and the image data acquired by this method corresponds to first image data in the present invention.

The reconstruction unit 730 generates the first image data by performing the first image generation method with use of the signal data read out from the storage unit 710, and stores the generated image data into the storage unit 710. In the present exemplary embodiment, the first image generation method will be described referring to an example in which the universal back-projection (UBP) reconstruction, which is an analytical image reconstruction method, is employed as the first image generation method. Generally, the analytical image reconstruction method is a method that can reconstruct an image with use of an equation acquired by analytically solving the following photoacoustic wave equation.

$$p(r_0, t) = \frac{1}{4\pi c^2} \frac{\partial}{\partial t} \left[ \frac{1}{ct} \int dr \cdot p_0(r)\delta(t - \frac{|r - r_d|}{c}) \right] \quad \cdots \quad (1)$$

In this equation, $p(r_0, t)$ represents an ideal photoacoustic signal detected at a position $r_0$ of a detector at a time t, c represents an acoustic speed, and $p_0(r)$ represents an initial acoustic pressure distribution. Further, $\delta$ represents a delta function. As the analytical image reconstruction method, there are various methods, specific examples of which include the filtered back-projection method, the Fourier transform method, and the like. For example, the method called UBP can be expressed by the following equation.

$$p_0(r) = \frac{2}{\Omega_0} \int_{S_0} \left[ p_d(r_0, t) - t \frac{\partial p_d(r_0, t)}{\partial t} \right]_{t = \frac{|r - r_0|}{c}} d\Omega_0 \quad \dots \quad (2)$$

In this equation, $S_0$ represents a detection surface surrounding an acoustic source, and $\Omega_0$ represents a solid angle and is 2n in a semi-infinite plane and 4n in an infinite cylinder and a spherical surface. Further, $d\Omega_0$ represents a solid angle of a detector $dS_0$ with respect to an arbitrary observation point P. The initial acoustic pressure distribution $P_0(r)$ can be acquired by carrying out back-projection of projection data indicated in [ ] according to integration of the equation (2).

The use of the analytical image reconstruction method allows the photoacoustic apparatus to acquire image data in which the acoustic source is completely reproduced when ideal reception elements (capable of detecting all bands and points) are arranged on an infinite plane, an infinite cylinder, or a spherical surface without any space left.

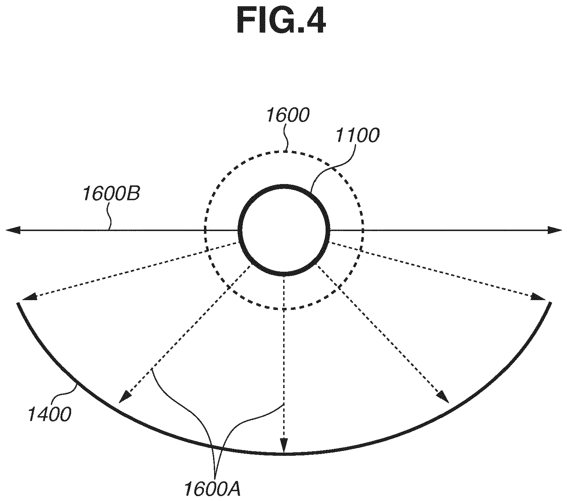

On the other hand, if the image is analytically reconstructed with use of the signal from the reception unit 400 in which the reception element group 410 is arranged on the semispherical support member 420 as illustrated in Fig. 1, the acoustic source cannot be reproduced. A reason therefor will be described with reference to a result of a simulation using a model illustrated in Fig. 4. In the present simulation, a model therefor is hypothesized in such a manner that a reception unit 1400 including a reception element group arranged on a semi-sphere receives a photoacoustic wave generated from a ring-shaped optical absorber 1100 (acoustic source model). In this case, an acoustic wave 1600 is propagated from the ring-shaped optical absorber 1100 in every direction over 360 degrees. The acoustic wave 1600 propagated in a direction 1600A is received by the reception unit 1400, but the acoustic wave 1600 propagated in a direction 1600B is not received by the reception unit 1400. As a result, in the analytical image reconstruction method, a region where the acoustic wave 1600 has not been received is regarded as being a region where there is a reception element that has not received the acoustic wave 1600. Further, this leads to a failure in imaging the acoustic source in the reconstructed image despite the fact that the acoustic wave 1600 is generated from the acoustic source.

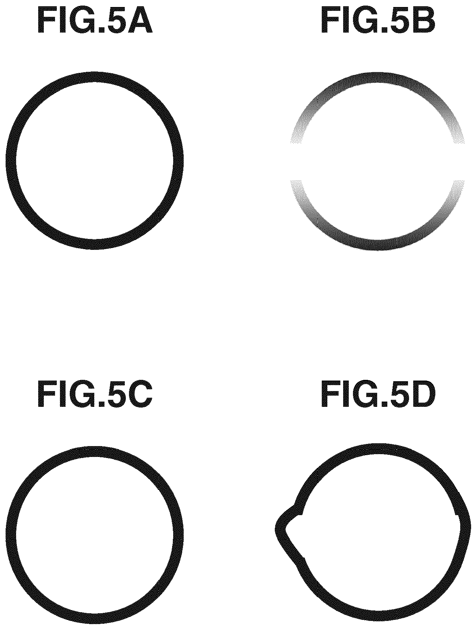

Fig. 5A illustrates the optical absorber 1100, which is the acoustic source model also illustrated in Fig. 4. Further, Fig. 5B illustrates the first image data of the optical absorber 1100 that is acquired by the analytical image reconstruction method (the UBP reconstruction) with use of the signal output from the reception unit 1400. As understood from Fig. 5B, the image cannot be visibly confirmed at a central portion of the optical absorber 1100 in the image acquired by the UBP reconstruction. This phenomenon is called a Limited View problem, and a condition under which the image of the actual acoustic source cannot be acquired with use of the analytical image reconstruction is called a Limited View condition. However, the image data acquired by the analytical image reconstruction can serve as highly reliable data regarding the clearly imaged structure. This is because a degree of estimation when the image data is acquired is small compared to the image generation method using the information regarding the structure of the subject 1000, which will be described below.

The image generation method for acquiring the first image data has been described referring to the analytical image reconstruction by way of example, but the first image data may be generated by any image generation method as long as this method is the image generation method that does not use the information regarding the structure of the subject 1000, which will be described below. For example, methods employable as the image generation method for acquiring the first image data include a time reversal method that does not take into consideration the information regarding the structure of the subject 1000, a model-based method including a Tikhonov regularization term, and the like.

<Step S200: Process for Generating Image Data based on Information regarding Structure of Subject 1000>

In this process, the computer 700 performs the image data generation method for generating the image data based on the information regarding the structure of the subject 1000. This image generation method corresponds to a second image generation method in the present exemplary embodiment, and the image data acquired by this method corresponds to second image data in the present exemplary embodiment. Such image generation methods will be described in detail below.

In this process, the reconstruction unit 730 may generate the second image data by performing the image generation method based on the information regarding the structure of the subject 1000 with use of the signal data read out from the storage unit 710. In other words, the reconstruction unit 730 may carry out the reconstruction based on the information regarding the structure of the subject 1000. Alternatively, the image processing unit 750 may perform the image generation method for generating the second image data based on the first image data acquired in step S100 and the information regarding the structure of the subject 1000. The second image data acquired in this manner is stored into the storage unit 710. Each of the image generation methods will be described below.

<Reconstruction based on Information regarding Structure of Subject 1000>

First, the information regarding the structure of the subject 1000 will be defined. The information regarding the structure of the subject 1000 according to the present exemplary embodiment refers to characteristic information derived from the characteristic structure of the measurement target inside the subject 1000. For example, if the measurement target is the blood vessel, the information regarding the structure of the subject 1000 refers to a structural characteristic of this blood vessel. These kinds of characteristic information are also called pre-acquired information because it is able to be known before the measurement.

In the reconstruction using the information regarding the structure of the subject 1000, for example, solving an equation (3) can achieve the reconstruction in which the characteristic information derived from the characteristic structure of the measurement target inside the subject 1000 is introduced.

$$ E = \arg\min_{p_0}\{\|p_d - p_c\|^2 + \lambda f(p_0)\} \qquad \ldots\ (3) $$

In this equation, E represents a cost function. Further, $p_d$ represents the signal (the time-resolved signal) output from each of the reception elements 411 to 414 of the reception unit 400. Further, $p_c$ represents a signal acquired from a numerical calculation (simulation) as a signal output from each of the reception elements 411 to 414 due to the acoustic wave that is, when the acoustic source distribution (the initial acoustic pressure distribution) data in the subject 1000 is arbitrarily hypothesized, generated therefrom. Further, $p_0$ represents the above-described hypothesized acoustic source distribution data.

A first term on a right-hand side of the equation (3) is a least square term. First, the photoacoustic signal ($p_c$) that should be acquired at each reception element position in the arbitrarily hypothesized acoustic source distribution ($p_0$) in the subject 1000 is estimated from the simulation. Next, the estimated hypothetical signal ($p_c$) and the actually measured signal ($p_d$) acquired from the measurement are compared to each other. Then, if the hypothetical signal and the actually measured signal match each other with a difference therebetween falling within a predetermined numerical range, the acoustic source distribution ($p_0$) hypothesized at this time is determined to be an optimum solution ($\hat{p}_0$). The first term on the right-hand side of the equation (3) is a term for these functions.

A second term on the right-hand side, $f(p_0)$ is called a regularization term, a constraint term, or a penalty term. The regularization term is a term for imposing a constraint on the solution of the least square cost function that is the term previous thereto, thereby narrowing down candidates to a further appropriate solution. Further, $\lambda$ is an arbitrary constant and a value for balancing the least square term and the regularization term, and is normally a preset value. In this case, the hypothesized acoustic source distribution $p_0$ is set as a variable in the function (the regularization term) defined at the second term on the right-hand side. Such an acoustic source distribution ($p_0$) in the subject 1000 that the cost function E including a value evaluated by this function is minimized as much as possible is determined to be a candidate for the optimum solution.

In other words, the reconstruction unit 730 can acquire the second image data by carrying out model-based reconstruction that solves the cost function including the difference between the actually measured signal and the hypothetical signal, and the regularization term based on the information regarding the structure of the subject 1000, with use of the optimization method.

The regularization term according to the present exemplary embodiment is a term set by the reconstruction unit 730 based on the information regarding the structure of the subject 1000. For example, suppose that light of 400 to 1100 nm is used when the photoacoustic imaging is applied to the living body. In this case, an absorption coefficient of hemoglobin is typically higher than another composition such as fat and water, so that a blood vessel (blood) including a large amount of hemoglobin is imaged. Therefore, it is desirable to determine a solution including a large number of characteristics of a vascular structure as the solution to the optimization problem (optimum estimated solution). In other words, the regularization term according to the present exemplary embodiment is a regularization term imposing such a constraint that the solution including a large number of characteristic structures of the subject 1000 like the vascular structure is acquired as the optimum solution.

As a method for determining the regularization term, the regularization term may be determined based on machine learning of the characteristic structure of the subject 1000. Regarding the vascular structure, it is known that a three-dimensional vascular structure can be imaged by X-ray computerized tomography (CT) or magnetic resonance imaging (MRI) using a contrast agent. By imaging various blood vessels with use of CT or MRI and applying a signal processing technique thereto, a basis image ($\varphi$) describing the characteristic of the vascular image can be generated from this captured image. Examples of the method therefor include the principal component analysis, the independent principal component analysis, the nonnegative matrix factorization, the sparse signal decomposition. Once the basis image describing the characteristic of the image can be acquired by such a method, a vascular image $p_0$, vessel, which is acquired in the photoacoustic imaging, can be approximately expressed as indicated by the following equation (4) with use of the basis image.

$$p_{0,\ vessel} = a_0\emptyset_0 + a_1\emptyset_1 + a_2\emptyset_2 + \dots + a_n\emptyset_n = \sum_{i=0}^{n} a_i\emptyset_i \ \dots \ (4)$$

In this equation, $a_i$ (i = 0 ... n) represents a coefficient for each basis. Assuming that $\Phi = (\varphi_0, ..., \varphi_n)$ represents a basis matrix in which acquired bases are arranged, and a = ($a_0$, $a_1$, ... $a_n$) represents a coefficient vector in which acquired coefficients are arranged, the equation (4) can be expressed as $p_{0,\ vessel} = \Phi a$ with use of the matrix.

Normally, the number n of basis vectors and the dimension of the image coincide with each other, but the number n of basis vectors can also be made larger than the dimension of the image and such a case is called an overcomplete basis matrix. In such a case, the basis may be called a frame. When the vascular image $p_0$, vessel acquired in the photoacoustic imaging is decomposed with the overcomplete basis matrix, the coefficient vector is not uniquely determined. Further, generating the basis (frame) of the image with use of the method that is called the sparse signal decomposition can lead to generation of such a basis that only a small number of coefficients $a_n$ in the coefficient vector a have a non-zero value, and most of the remaining coefficients become zero. Use of the basis generated by such a method allows the image to be efficiently expressed with a small number of bases. In other words, making the coefficient vector redundant with the aid of the overcomplete basis matrix and also expressing the image with the small number of bases allow the image to be expressed as a further excellent approximated image, and also enhance robustness against a noise.

In the present exemplary embodiment, the reconstruction unit 730 also sets the regularization term for estimating the solution in which the image can be efficiently expressed with the generated basis $\varphi$, thereby allowing the acoustic source distribution (the vascular structure) to be accurately reproduced. For example, if it is assumed that the overcomplete basis matrix $\varphi$ is generated with the sparse signal decomposition and the vascular image is constructed with as few bases as possible (a sparse expression), the intended solution can be acquired by minimizing the cost function E expressed as the equation (5).

$$\hat{p}_0 = \arg\min_{p_0}\{\|p_d - Ap_0\|^2 + \lambda\|a\|_1\} \ \dots \ (5)$$

In other words, the reconstruction unit 730 removes unnecessary information by imposing the constraint that the image is constructed with the small number of bases, which further facilitates the reconstruction of the image including the characteristic of the blood vessel. In the present exemplary embodiment, the image generation method has been described referring to the example in which the blood vessel is imaged by the photoacoustic imaging, but a structure of a tissue other than the blood vessel can be imaged depending on the wavelength of the light 130 to be used and the site to be measured. For example, plaque including a large amount of fat and a tissue including a large amount of melanin can be imaged. In such a case, the above-described method can be applied by using a basis that can express a characteristic of the plaque including the large amount of fat, the tissue including the large amount of melanin, or the like.

Now, a specific calculation example will be described below. For example, the equation (1) can be expressed by the following equation, by being spatially discretized and expressed in the form of a matrix.

$$p_d = Ap_0 \ \dots \ (6)$$

In this equation, $p_d$ represents a column vector expressing the signal output from each of the reception elements 411 to 414. Further, $p_0$ represents a column vector expressing the discretized acoustic source distribution (the initial acoustic pressure distribution). Further, A represents a forward model matrix (an operator expressed in the form of a matrix) expressing that the photoacoustic wave generated from some extremely small acoustic source is received by the reception element 411, 412, 413, or 414 and is converted into the signal. In practice, a forward model matrix that correctly models the propagation of the photoacoustic wave and a reception characteristic of each of the reception elements 411 to 414 is generated. For this purpose, the forward model matrix A is generated in advance in consideration of the reception characteristic of each of the reception elements 411 to 414, such as a size effect (directionality) and an impulse response of each of the reception elements 411 to 414, the pulse width of the incident light, reflection and attenuation of the photoacoustic wave, and the like, and is stored into the storage unit 710. For example, a group of signals that the respective reception elements 411 to 414 of the reception unit 400 output by receiving the photoacoustic waves generated due to the irradiation of the subject 1000 with the light 130 is stored into the storage unit 710 as the column vector ($p_d$). Then, if the matrix A is a regular matrix, the matrix A has an inverse matrix, so that the initial acoustic pressure distribution $p_0$ can be expressed by an equation (7).

$$p_0 = A^{-1} p_d \ \dots \ (7)$$

In this equation, $A^{-1}$ represents the inverse matrix of the matrix A. As understood from this equation, the acoustic source distribution $p_0$ can be acquired by multiplying the received acoustic pressure vector by the inverse matrix of the matrix A. Conversely, the reconstruction unit 730 can acquire $p_c$ in the equation (3) by multiplying the hypothesized acoustic source distribution (matrix vector) $p_0$ by the forward model matrix A.

However, when the semispherical reception unit 400 configured as illustrated in Fig. 1 is used, this case corresponds to the Limited View condition as described above, so that the matrix A is not the regular matrix. Therefore, the matrix A does not have the inverse matrix. In this case, the reconstruction unit 730 solves the conditioned optimization problem for acquiring $p_0$ that minimizes the cost function E expressed as indicated by the equation (3).

Now, as the characteristic of the vascular structure, there is Murray's law, which is known as a vascular bifurcation model. The reconstruction unit 730 may set the regularization term based on the information regarding the structure of the subject 1000 according to Murray's law. According to Murray's law, a relationship expressed by an equation (8) is established, assuming that $r_0$ represents a vascular width before a bifurcation, and $r_1$ and $r_2$ represent vascular widths after the bifurcation, respectively.

$$r_0^3 = r_1^3 + r_2^3 \ \dots \ (8)$$

Further, assuming that $\theta_1$ and $\theta_2$ represent bifurcation angles after the bifurcation, respectively, relationships expressed by equations (9) and (10) are established.

$$\cos \theta_1 = \frac{r_0^4 + r_1^4 - r_2^4}{2 r_0^2 r_1^2} \ \dots \ (9)$$

$$\cos \theta_2 = \frac{r_0^4 + r_2^4 - r_1^4}{2 r_0^2 r_2^2} \ \dots \ (10)$$

In other words, the frame (d), which is the redundant basis expressing the characteristic structure of the blood vessel, can be generated by numerically generating various vascular images with use of Murray's law, and carrying out the machine learning of these pieces of image data. In the present exemplary embodiment, this is used as the information regarding the structure of the subject 1000. Then, assuming that $D = (d_0, ..., d_n)$ represents a frame matrix (dictionary) and $\alpha = (\alpha_1, ..., \alpha_n)$ represents a coefficient vector, the estimated acoustic source distribution $p_0$ is expressed by the following equation (11).

$$p_0 = D\alpha \ \dots \ (11)$$

In other words, the acoustic source distribution $p_0$ can be expressed by a combination of the frames (d) in the dictionary D, which is the information regarding the structure of the subject 1000. In such a case, assuming that the acoustic source distribution $p_0$ is constructed with a further small number of frames (sparse expression), the optimum acoustic source distribution $p_0$ can be acquired by solving the following equation (12).

$$E = \arg \min_{\alpha} \left\{ \left\| p_d - A \cdot p_0 \right\|^2 + \lambda \left\| \alpha \right\|_1 \right\} \ \dots \ (12)$$

A constraint that the optimum acoustic source distribution is constructed with a minimum number of frames may be introduced.

The reconstruction unit 730 can acquire the image data illustrated in Fig. 5C by carrying out this reconstruction on the signal data derived from the photoacoustic wave generated from the ring-shaped optical absorber 1100 illustrated in Fig. 5A. In other words, the reconstruction unit 730 can form an image closer to Fig. 5A than to Fig. 5B by this reconstruction. However, in such a case that the structure indicated by the information regarding the structure of the subject 1000 and the structure of the subject 1000 do not match each other, the ring-shaped optical absorber 1100 illustrated in Fig. 5A may be reconstructed as illustrated in Fig. 5D. In such a case, presenting only the image data illustrated in Fig. 5D to the user does not allow the user to determine which region is a highly reliable region.

The reconstruction method using the information regarding the structure of the subject 1000 is not limited to the reconstruction method using the sparse expression described herein, and may be any reconstruction method as long as this method is the reconstruction capable of introducing an evaluation of how much the characteristic structure of the subject 1000 is contained in the hypothesized acoustic source distribution.

<Processing for Correcting Image based on Information regarding Structure of Subject 1000>

This method is a method in which the image processing unit 750 corrects the first image data acquired by the reconstruction unit 730 in step S100 by performing image processing based on the information regarding the structure of the subject 1000. First, the image processing unit 750 reads out the first image data stored in the storage unit 710. Although having also been described above, the first image data in which the shape of the acoustic source cannot be completely reproduced as illustrated in Fig. 5B, is acquired by the apparatus configured as illustrated in Fig. 1 under the Limited View condition. Assume that g represents such first image data in which the shape of the acoustic source cannot be completely reproduced, and g~ represents corrected image data. The image processing method using the information regarding the structure of the subject 1000 according to the present exemplary embodiment can be formulated as, for example, the following equation (13).

$$E = \arg\min_{\widetilde{g}}\{\|g - \widetilde{g}\|^2 + \lambda \cdot f(\widetilde{g})\} \quad \cdots \ (13)$$

In this equation, g~ represents optimum corrected image data. A first term on a right-hand side is a least square term, and f(g~) and $\lambda$ in a second term represent a regularization term and a parameter indicating a weight allocated to the regularization term, respectively. The reconstruction using the information regarding the structure of the subject 1000 is the method that hypothesizes the acoustic source distribution $p_0$ with respect to the signal data and solves the optimization problem for optimizing the cost function including the difference between the actually measured signal and the hypothetical signal. On the other hand, this image processing acquires an optimum solution of the corrected image data g~ by hypothesizing the corrected image data g~ with respect to the first image data and solving an optimization problem for optimizing a cost function including a difference between the first image data and the corrected image data (hypothetical image data). The image processing unit 750 can set a term similar to the regularization term $f(p_0)$ in the reconstruction using the information regarding the structure of the subject 1000 as the regularization term f(g~).

The image processing unit 750 can correct the first image data illustrated in Fig. 5B into the corrected image data (second image data) illustrated in Fig. 5C, by performing this image processing. However, in such a case that the structure indicated by the information regarding the structure of the subject 1000 and the structure of the subject 1000 do not match each other, the image may be reconstructed as illustrated in Fig. 5D. In such a case, presenting only the image data illustrated in Fig. 5D to the user does not allow the user to determine which region is a highly reliable region.

The image processing using the information regarding the structure of the subject 1000 is not limited to the image processing using the sparse expression, and may be any method as long as this method is the image processing method capable of introducing the evaluation of how much the characteristic structure of the subject 1000 is contained in the first image data.

As described above, in the present exemplary embodiment, the computer 700 generates the second image data based on the signal acquired from the reception unit 400 and the information regarding the structure of the subject 1000.

<Step S300: Process for Displaying Second Image Data>

The control unit 770 as the display control unit reads out the second image data acquired in step S200 from the storage unit 710, transfers the read image data to the display unit 800, and causes the transferred image data to be displayed on the display unit 800. However, this second image data has been estimated with use of the information regarding the structure of the subject 1000. Therefore, the second image data may include a region formed only from the information regarding the structure of the subject 1000 without being reconstructed based on the signal from the reception unit 400 depending on a location in the image data, thereby including a low reliability region. Further, displaying only the second image data does not allow the user to determine whether the reliability is high or low at each position in the displayed image data.

<Step S400: Process for Displaying Second Image Data Distinguishably between Difference Region between First Image Data and Second Image Data, and Another Region>

The control unit 770 as the display control unit causes the second image data to be displayed on the display unit 800 so as to be able to distinguish a difference region between the first image data and the second image data, and another region from each other. Causing the second image data to be displayed in this manner makes it possible to distinguish the region estimated derivatively from the information regarding the structure of the subject 1000 and the highly reliable region derived from the signal output from the reception unit 400 in the second image data from each other.

For example, the control unit 770 can extract the difference region with use of the first image data and the second image data, and cause this region to be displayed by a different display method. First, the control unit 770 reads out the first image data and the second image data from the storage unit 710, and generates difference image data indicating a difference between the first image data and the second image data. The control unit 770 identifies a pixel or voxel included in a predetermined numerical range among image values of the difference image data, and sets the identified pixel or voxel as the difference region. For example, the control unit 770 identifies a pixel or voxel exceeding a predetermined threshold value among the image values of the difference image data, and sets the difference region. Then, the control unit 770 changes a method for displaying the pixel or voxel included in the difference region and a method for displaying a pixel or voxel included in another region in the second image data, thereby causing each of the regions to be displayed distinguishably from each other. For example, the control unit 770 can cause the second image data to be displayed so as to be able to distinguish the regions in the second image data from each other by, for example, changing a color or changing whether to present the display in a blinking manner for each region.

Further or alternatively, the control unit 770 may cause the second image data to be displayed on the display unit 800 while an image value of the first image data is associated with at least one of a hue and saturation, and an image value of the second image data is associated with brightness. This display allows the user to confirm the highly reliable region defined by the first image data and the low reliability region missing in the first image data in the second image data based on different colors.

Further or alternatively, the control unit 770 can cause the first image data and the second image data to be displayed while being superimposed with different hues allocated to them. At this time, a region where the first image data and the second image data overlap each other may be displayed in the color allocated to the highly reliable first image data. Displaying the image based on the highly reliable image data facilitates distinguishing the low reliability region that may lead to a misdiagnosis. Alternatively, the region where the first image data and the second image data overlap each other may be displayed with a different hue from any of the hues allocated to the first image data and the second image data. If the first image data and the second image data are displayed on the display unit 800 with the change made between the methods for displaying the first image data and the second image data, it is desirable to superimpose the images to facilitate a comparison between the images.

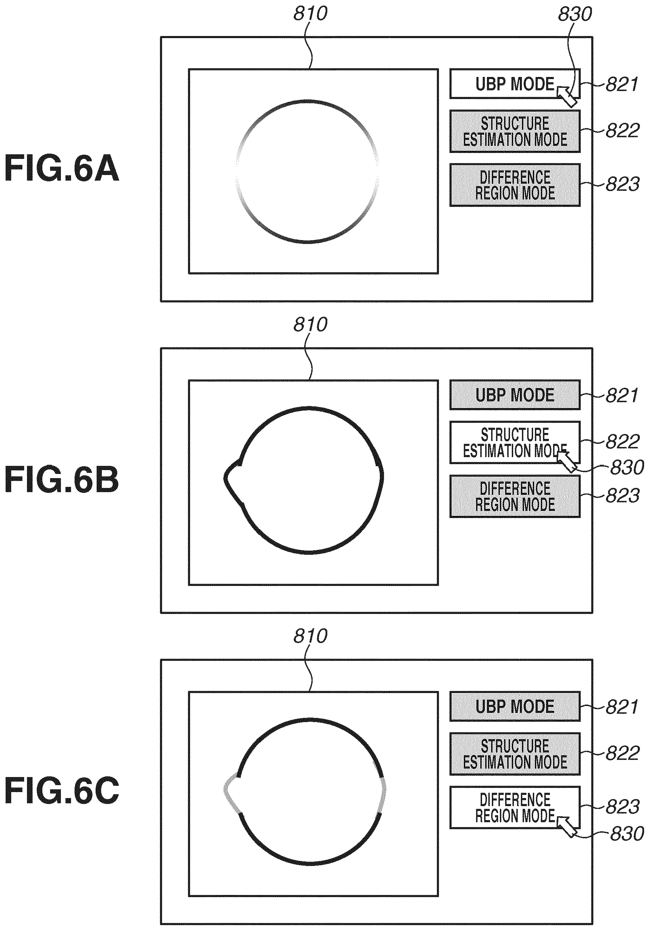

Figs. 6A, 6B, and 6C each illustrate an example of a GUI displayed on the display unit 800. A display screen 810, a UBP icon 821, a structure estimation icon 822, a difference region icon 823, and an arrow icon 830 are displayed on the GUI. The user can move the arrow icon 830 by operating the input unit 900, and select the various kinds of icons 821, 822, and 823.

Fig. 6A illustrates the GUI when the UBP icon 821 is selected. When the UBP icon 821 is selected, the control unit 770 reads out the first image data from the storage unit 710, and causes the read first image data to be displayed on the display screen 810. In other words, the UBP icon 821 is associated with the first image data.

Fig. 6B illustrates the GUI when the structure estimation icon 822 is selected. When the structure estimation icon 822 is selected, the control unit 770 reads out the second image data from the storage unit 710, and causes the read second image data to be displayed on the display screen 810. In other words, the structure estimation icon 822 is associated with the second image data.

Fig. 6C illustrates the GUI when the difference region icon 823 is selected. When the difference region icon 823 is selected, the control unit 770 reads out the first image data and the second image data from the storage unit 710. Then, the control unit 770 causes the second image data to be displayed on the display screen 810 so as to make the difference region and the other region distinguishable from each other by the method described in the description of step S400. Herein, the second image data is displayed with the color changed between the difference region and the other region. In other words, the difference region icon 823 is associated with both the first image data and the second image data.

As described above, the control unit 770 performs control so as to switch a mode of displaying the first image data, a mode of displaying the second image data, and a mode of displaying the second image data so as to make the difference region distinguishable. The photoacoustic apparatus can switch the displayed image data in this manner, thereby allowing the user to make a diagnosis while determining the reliability of the displayed information comprehensively from the plurality of images.

The labels added to the icons illustrated in Figs. 6A, 6B, and 6C may be any labels. Further, the method for selecting each of the display modes is not limited to the method using the arrow icon 830 and the like on the GUI, and may be any method as long as this method can switch the display modes. For example, the display mode may be switched via the input unit 900 realized by a hardware device such as a button, or may be switched by the control unit 770 automatically.

Further, the photoacoustic apparatus may be an apparatus including only the mode of displaying the second image data so as to make the difference region distinguishable. Even in this case, the photoacoustic apparatus can display the image data so as to be able to distinguish the region estimated derivatively from the information regarding the structure of the subject 1000.

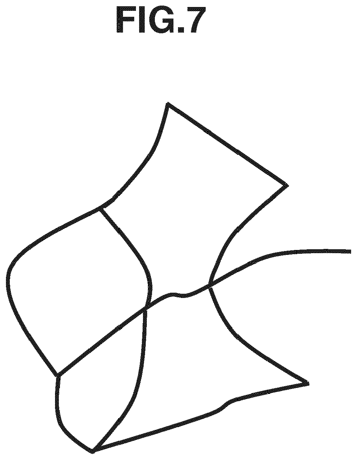

A second exemplary embodiment of the present invention will be described below. In the present exemplary embodiment, a degree of estimation of the structure of the subject can be changed when displaying the second image data. In the present exemplary embodiment, a case is described where the photoacoustic apparatus described in the first exemplary embodiment acquires vascular image data illustrated in Fig. 7.

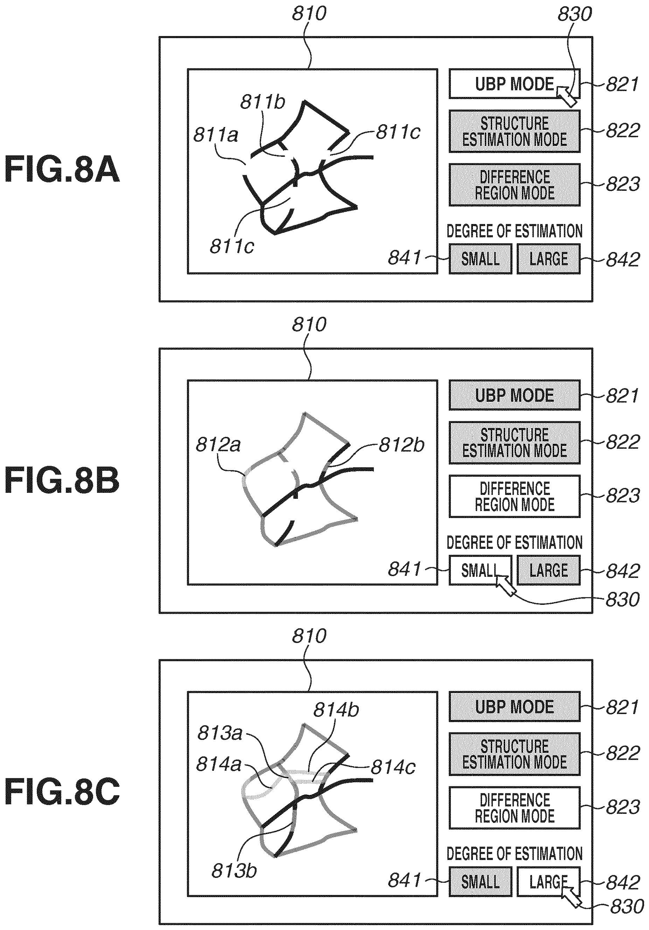

Figs. 8A, 8B, and 8C each illustrate an example of a GUI displayed on the display unit 800 according to the present exemplary embodiment. Similar components to those in Figs. 6A, 6B, and 6C will be denoted by the same reference numerals, and descriptions thereof will be omitted. A small degree estimation icon 841 and a large degree estimation icon 842 are displayed on each of the GUIs illustrated in Figs. 8A, 8B, and 8C. These icons 841 and 842 are icons associated with $\lambda$ indicated in the equations (3), (5), (12), and (13). The small degree estimation icon 841 is an icon associated with $\lambda$ having a small value, and the large degree estimation icon 842 is an icon associated with $\lambda$ having a large value. If the small degree estimation icon 841 is selected, this selection leads to a calculation with use of $\lambda$ having the small value. On the other hand, if the large degree estimation icon 842 is selected, this selection leads to a calculation with use of $\lambda$ having the larger value than that when the small degree estimation icon 841 is selected. In other words, in the present exemplary embodiment, the user can set the degree of estimation of the image data based on the information regarding the structure of the subject.

Fig. 8A illustrates the GUI when the UBP icon 821 is selected. Comparing the first image data displayed on the display screen 810 illustrated in Fig. 8A with blood vessels that are the measurement target illustrated in Fig. 7, it is understood that blood vessels in regions 811a, 811b, 811c, and 811d have failed to be reproduced. As illustrated in Fig. 8A, when the UBP icon 821 associated with the first image data is selected, the icons 841 and 842 are disabled and are not selectable.

Fig. 8B illustrates the GUI when the difference region icon 823 and the small degree estimation icon 841 are selected. When the small degree estimation icon 841 is selected, the reconstruction unit 730 or the image processing unit 750 reads out $\lambda$ corresponding to the small degree estimation icon 841 that is stored in the storage unit 710, and generates the second image data. Then, the control unit 770 displays the second image data by the method described in the description of step S400 with use of the second image data acquired with use of $\lambda$ corresponding to the small degree estimation icon 841. As illustrated in Fig. 8B, structures 812a and 812b can be estimated based on $\lambda$ corresponding to the small degree estimation icon 841.

Fig. 8C illustrates the GUI when the difference region icon 823 and the large degree estimation icon 842 are selected. When the large degree estimation icon 842 is selected, the reconstruction unit 730 or the image processing unit 750 reads out $\lambda$ corresponding to the large degree estimation icon 842 that is stored in the storage unit 710, and generates the second image data. Then, the control unit 770 displays the second image data by the method described in the description of step S400 with use of the second image data acquired with use of $\lambda$ corresponding to the large degree estimation icon 842. As illustrated in Fig. 8C, structures 813a and 813b, and structures 814a, 814b, and 814c can be estimated based on $\lambda$ corresponding to the large degree estimation icon 842. Comparing the blood vessels illustrated in Fig. 7 and the image data illustrated in Fig. 8C, the correct reproduction of the blood vessel has succeeded regarding the structures 813a and 813b. However, the structures 814a, 814b, and 814c are structures not existing in the blood vessels that are the measurement target illustrated in Fig. 7, and are false images generated from the estimation.

As also understood from them, there is such a tendency that, as the degree of estimation of the structure is increased, a possibility of estimating the structure of the subject not existing in the first image data is increased. On the other hand, when the degree of estimation is increased, a possibility of falsely estimating the actually absent structure of the subject is also increased. Therefore, the photoacoustic apparatus can cause the image data of the structure to be displayed in conformity with the user's need, by allowing the user to set the degree of estimation via the input unit 900, like the present exemplary embodiment. For example, if the structure of the subject can be sufficiently extracted even in the first image data, the number of false images can be reduced by setting a small degree of estimation. On the other hand, if the structure of the subject cannot be sufficiently extracted in the first image data, a large degree of estimation can be set according to the user's determination so as to increase the number of extracted structures while even risking the possibility of the false image.

In the present exemplary embodiment, setting the degree of estimation has been described referring to the example in which the degree of estimation is selected from the two discontinuous large and small degrees of estimation via the input unit 900, but the degree of estimation may be specified by any method via the input unit 900. For example, the input unit 900 may be configured to allow the user to continuously change $\lambda$ corresponding to the degree of estimation.

It is considered that a normal blood vessel and an abnormal blood vessel generated around a tumor or the like are different from each other in terms of the characteristic structure thereof. Therefore, the structure of the abnormal blood vessel cannot be correctly reproduced from the estimation of the structure based on the information regarding the structure of the normal blood vessel. Conversely, the structure of the normal blood vessel cannot be correctly reproduced from the estimation of the structure based on the information regarding the structure of the abnormal blood vessel. Therefore, in a third exemplary embodiment of the present invention, a case is described where a mode of estimating the structure based on the characteristic structure of the normal blood vessel, and a mode of estimating the structure based on the characteristic structure of the abnormal blood vessel can be changed when displaying the second image data. In the present exemplary embodiment, suppose that the image data is acquired by the photoacoustic apparatus described in the description of the first exemplary embodiment.

In the present exemplary embodiment, the storage unit 710 stores a regularization term set so as to optimize the cost function when a large number of structures of the normal blood vessel are included, and a regularization term set so as to optimize the cost function when a large number of structures of the abnormal blood vessel are included. Further, the input unit 900 is configured to allow the user to specify a normal blood vessel estimation mode or an abnormal blood vessel estimation mode. The normal blood vessel estimation mode is associated with the regularization term set so as to optimize the cost function when the large number of structures of the normal blood vessel are included. The abnormal blood vessel estimation mode is associated with the regularization term set so as to optimize the cost function when the large number of structures of the abnormal blood vessel are included.

Then, if the user selects the normal blood vessel estimation mode with use of the input unit 900, the reconstruction unit 730 or the image processing unit 750 reads out the regularization term corresponding to the normal blood vessel estimation mode that is stored in the storage unit 710, and generates the second image data. On the other hand, if the user selects the abnormal blood vessel estimation mode with use of the input unit 900, the reconstruction unit 730 or the image processing unit 750 reads out the regularization term corresponding to the abnormal blood vessel estimation mode that is stored in the storage unit 710, and generates the second image data.

In this manner, the photoacoustic apparatus can switch the estimation modes corresponding to a plurality of types of vascular structures, thereby achieving the estimation suitable for the blood vessel that the user pays attention to. A possible procedure in this case is as follows. For example, if the abnormal blood vessels account for a small percentage of the entire image relative to the normal blood vessels, first, the photoacoustic apparatus confirms how the blood vessels run overall in the normal blood vessel estimation mode. Then, upon detecting a region seeming to include an abnormal blood vessel from these blood vessels, the photoacoustic apparatus switches the normal blood vessel estimation mode to the abnormal blood vessel estimation mode, and observes the region seeming to be the abnormal blood vessel. In this case, it is desirable that the normal blood vessel estimation mode is set as an initial setting when the structure estimation mode or the difference region mode is selected.

In the case where the regeneration of the second image data is specified interactively, like the second exemplary embodiment and the third exemplary embodiment, the second image data may be unable to be redisplayed in real time if being reconstructed based on the information regarding the structure of the subject. In such a case, it is desirable that the computer 700 performs the image correction processing based on the information regarding the structure of the subject, a processing amount of which is smaller compared to the reconstruction based on the information regarding the structure of the subject. Displaying the second image data in real time here means that the second image can be displayed within 0.1 seconds from issue of a regeneration instruction from the user.

Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

**Claims**

1. A photoacoustic apparatus comprising:

   a light irradiation means configured to irradiate a subject with light;
   a reception means configured to convert an acoustic wave generated from the subject due to the irradiation with the light into an electric signal;
   a processing means configured to acquire image data based on the electric signal; and
   a display control means,

   wherein the processing means acquires first image data by a first image generation method based on the electric signal, and acquires second image data by a second image generation method different from the first image generation method based on the electric signal, and

   wherein the display control means sets, as a difference region, a region in which difference image data between the first image data and the second image data has an image value falling within a predetermined numerical range, and causes image data in which the difference region and another region are distinguishable from each other to be displayed on a display means based on the first image data and the second image data.

2. The photoacoustic apparatus according to claim 1, wherein the processing means acquires the first image data based on the electric signal without use of information regarding a structure of the subject, and acquires the second image data based on the electric signal and the information regarding the structure of the subject.

3. The photoacoustic apparatus according to claim 2, wherein the processing means acquires the second image data by carrying out model-based reconstruction that solves a cost function including a difference between the electric signal and a hypothetical signal, and a regularization term based on the information regarding the structure of the subject, with use of an optimization method.

4. The photoacoustic apparatus according to claim 2, wherein the processing means acquires third image data based on the electric signal, and acquires the second image data by correcting the third image data based on the information regarding the structure of the subject.

5. The photoacoustic apparatus according to claim 4, wherein the processing means acquires the second image data by carrying out model-based reconstruction that solves a cost function including a difference between the third image data and hypothetical image data, and a regularization term based on the information regarding the structure of the subject, with use of an optimization method.

6. The photoacoustic apparatus according to any one of claims 2 to 5, wherein the information regarding the structure of the subject is information regarding a vascular structure.

7. The photoacoustic apparatus according to any one of claims 1 to 6, wherein the display control means causes the difference region and the other region to be displayed on the display means with different hues from each other.

8. The photoacoustic apparatus according to any one of claims 1 to 6, wherein the display control means causes the image in which an image value in the first image data is associated with at least one of a hue and saturation, and an image value in the second image data is associated with brightness, to be displayed on the display means.

9. The photoacoustic apparatus according to any one of claims 1 to 6, wherein the display control means causes the first image data and the second image data to be displayed on the display means in a first color and a second color different from the first color, respectively.

10. The photoacoustic apparatus according to claim 9, wherein the display control means causes a region where the first image data and the second image data overlap each other to be displayed on the display means in a third color different from the first color and the second color.

11. The photoacoustic apparatus according to claim 9, wherein the display control means causes a region where the first image data and the second image data overlap each other to be displayed on the display means in the first color associated with the first image data.

12. The photoacoustic apparatus according to any one of claims 1 to 6, wherein the display control means causes the difference region to be displayed on the display means in a blinking manner.

13. A display control method for image data acquired based on an electric signal derived from a photoacoustic wave generated from a subject due to irradiation of the subject with light, the display control method comprising:

displaying first image data acquired by a first image generation method based on the electric signal;
displaying second image data acquired by a second image generation method different from the first image generation method based on the electric signal, as a display of the second image data; and
setting, as a difference region, a region in which difference image data between the first image data and the second image data has an image value falling within a predetermined numerical range,

wherein the display of the second image data includes a mode of displaying the second image data so as to cause the difference region and another region to be distinguishable from each other.

14. The display control method according to claim 13, wherein the display of the second image data includes the mode of displaying the second image data so as to cause the difference region and the other region to be distinguishable from each other, and the mode of displaying the second image data so as not to cause the difference region and the other region to be distinguishable from each other.

15. A program for causing a computer to perform the display control method according to claim 14.

# FIG.1

SIGNAL DATA COLLECTION UNIT

STORAGE UNIT

IMAGE PROCESSING UNIT

RECONSTRUCTION UNIT

CONTROL UNIT

LIGHT SOURCE

INPUT UNIT

EP 3 178 380 A1

# FIG.2

700

701
CPU

702
GPU

703
RAM

704
ROM

705
EXTERNAL STORAGE DEVICE

801
LIQUID CRYSTAL DISPLAY

901
MOUSE

902
KEYBOARD

# FIG.3

```
        ┌─────────────┐
        │    START    │
        └─────────────┘
               │
               ▼                          ⌐S100
┌───────────────────────────────────────┐
│         GENERATE FIRST IMAGE DATA      │
│         WITHOUT USE OF INFORMATION     │
│       REGARDING STRUCTURE OF SUBJECT   │
└───────────────────────────────────────┘
               │
               ▼                          ⌐S200
┌───────────────────────────────────────┐
│        GENERATE SECOND IMAGE DATA      │
│      BASED ON INFORMATION REGARDING    │
│            STRUCTURE OF SUBJECT        │
└───────────────────────────────────────┘
               │
               ▼                          ⌐S300
┌───────────────────────────────────────┐
│          DISPLAY SECOND IMAGE DATA     │
└───────────────────────────────────────┘
               │
               ▼                          ⌐S400
┌───────────────────────────────────────┐
│   DISPLAY SECOND IMAGE DATA SO AS TO   │
│   CAUSE DIFFERENCE REGION BETWEEN      │
│   FIRST IMAGE DATA AND SECOND IMAGE    │
│     DATA, AND ANOTHER REGION TO BE     │
│   DISTINGUISHABLE FROM EACH OTHER      │
└───────────────────────────────────────┘
               │
               ▼
        ┌─────────────┐
        │     END     │
        └─────────────┘
```

# FIG.4

FIG.5A

FIG.5B

FIG.5C

FIG.5D

FIG.6A

FIG.6B

FIG.6C

# FIG.7

**FIG.8A**

810 830

811b
811a 811c
811c

UBP MODE — 821
STRUCTURE ESTIMATION MODE — 822
DIFFERENCE REGION MODE — 823
DEGREE OF ESTIMATION
841 — SMALL  LARGE — 842

**FIG.8B**

810

812a 812b

UBP MODE — 821
STRUCTURE ESTIMATION MODE — 822
DIFFERENCE REGION MODE — 823
DEGREE OF ESTIMATION
841 — SMALL  LARGE — 842
830

**FIG.8C**

810

814b
813a
814a 814c
813b

UBP MODE — 821
STRUCTURE ESTIMATION MODE — 822
DIFFERENCE REGION MODE — 823
DEGREE OF ESTIMATION
841 — SMALL  LARGE — 842
830

| Europäisches Patentamt European Patent Office Office européen des brevets | | **EUROPEAN SEARCH REPORT** | Application Number EP 16 20 1784 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/201135 A1 (OH JUNG-TAEK [KR] ET AL) 16 July 2015 (2015-07-16) | 1,2,4, 7-15 | INV. A61B5/00 |
| Y | * abstract; figures 3, 4, 6A-6C * * paragraphs [0010], [0019], [0051], [0094], [0101] - [0106] * * the whole document * | 3,5 | |
| X | US 2014/286549 A1 (FUKUTANI KAZUHIKO [JP]) 25 September 2014 (2014-09-25) | 1-15 | |
| Y | * abstract; figures 2, 5A, 5B * * paragraphs [0007], [0008], [0036] * * the whole document * & JP 2014 180492 A (CANON KK) 29 September 2014 (2014-09-29) | 3,5 | |
| X | SHUHUI BU ET AL: "Model-Based Reconstruction Integrated With Fluence Compensation for Photoacoustic Tomography", IEEE TRANSACTIONS ON BIOMEDICAL ENGINEERING, IEEE SERVICE CENTER, PISCATAWAY, NJ, USA, vol. 59, no. 5, 1 May 2012 (2012-05-01), pages 1354-1363, XP011490063, ISSN: 0018-9294, DOI: 10.1109/TBME.2012.2187649 | 1-15 | TECHNICAL FIELDS SEARCHED (IPC) A61B |
| Y | * abstract * * page 1354, right-hand column, paragraph second - page 1355, left-hand column, paragraph first * * the whole document * | 3,5 | |

The present search report has been drawn up for all claims

| Place of search | Date of completion of the search | Examiner |
|---|---|---|
| The Hague | 5 April 2017 | Furlan, Stéphane |

## ANNEX TO THE EUROPEAN SEARCH REPORT
## ON EUROPEAN PATENT APPLICATION NO.

EP 16 20 1784

This annex lists the patent family members relating to the patent documents cited in the above-mentioned European search report.
The members are as contained in the European Patent Office EDP file on
The European Patent Office is in no way liable for these particulars which are merely given for the purpose of information.

05-04-2017

| Patent document cited in search report | | Publication date | Patent family member(s) | | Publication date |
|---|---|---|---|---|---|
| US 2015201135 | A1 | 16-07-2015 | CN 104771136 A<br>EP 2893868 A1<br>KR 20150084559 A<br>US 2015201135 A1 | | 15-07-2015<br>15-07-2015<br>22-07-2015<br>16-07-2015 |
| US 2014286549 | A1 | 25-09-2014 | JP 2014180492 A<br>US 2014286549 A1 | | 29-09-2014<br>25-09-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2014180492 A